# EUROPEAN PATENT APPLICATION

(11) **EP 3 067 342 A1**
(43) Date of publication of application: **14.09.2016**
(21) Application number: 14860086.9
(22) Date of filing: 05.11.2014
(51) Int. Cl.: C07C 63/70, C07C 63/06, C07C 235/84, C07F 5/02, C07F 9/54, C07F 1/08

(54) **SUPERELASTIC MATERIAL AND ENERGY STORAGE MATERIAL, ENERGY ABSORPTION MATERIAL, ELASTIC MATERIAL, ACTUATOR, AND SHAPE MEMORY MATERIAL THAT USE SAID SUPERELASTIC MATERIAL**

(30) Priority: 05.11.2013 JP 2013229341
(71) Applicant: Yokohama City University, Yokohama-shi, Kanagawa 236-0027 (JP)
(72) Inventor: TAKAMIZAWA, Satoshi, Yokohama-shi Kanagawa 236-0027 (JP)
(74) Representative: Raynor, Stuart Andrew
(86) International application number: PCT/JP2014/079296
(87) International publication number: WO 2015/068712

(57) **Abstract**

A superelastic material is provided, which can exhibit superelasticity even with a reduced amount of energy compared with the conventional superelastic materials. The superelastic material is characterized by having a molecular crystal. The molecular crystal preferably has an organic skeleton.

## Description

### [Technical Field]

The present invention relates to a superelastic material and also to an energy storage material, an energy absorption material, an elastic material, an actuator, and a shape memory material that use the superelastic material.

### [Background Art]

Since an Au-Cd alloy was found to exhibit superelasticity in 1932 as disclosed in Non-Patent Literature 1, development of superelastic materials has been performed. In the present description, the "superelasticity" refers to a phenomenon that, when the applied external force is reduced, reverse transformation spontaneously occurs and this reverse transformation releases the energy stored during transformation thereby to finally recover a state that is substantially the same as the state before occurrence of the transformation.

Various superelastic materials have been developed, including metal-based materials as disclosed in Patent Literature 1 and oxide ceramic-based materials as disclosed in Patent Literature 2. In the present description, the "superelastic material" means a material that can exhibit superelasticity within a given temperature region.

### [Prior Art Literature]

### [Patent Literature]

[Patent Literature 1] JP2006-265680A
[Patent Literature 2] JP2004-149381A

### [Non-Patent Literature]

[Non-Patent Literature 1] Olander, A. An electrochemical investigation of solid cadmium-gold alloys. J. Am. Chem. Soc. 54, 3891-3833 (1932)

### [Disclosure of the Invention]

### [Problems to be solved by the Invention]

In the superelastic materials as disclosed in Patent Literature 1, 2, however, it is difficult to reduce the amount of energy required for the materials to exhibit the superelasticity. Therefore, when such superelastic materials are applied to microstructures such as MEMS (Micro Electro Mechanical Systems), the microstructures cannot appropriately exhibit a function based on the superelasticity, which may be problematic.

An object of the present invention is to provide a superelastic material that can exhibit superelasticity even with a reduced amount of energy compared with the conventional superelastic materials. Another object of the present invention is to provide an energy storage material, an energy absorption material, an elastic material, an actuator, and a shape memory material that use the above superelastic material.

### [Means for solving the Problems]

An aspect of the present invention, which is provided to achieve the above objects, is a superelastic material characterized by having a molecular crystal.

The molecular crystal of the above superelastic material may have an organic skeleton.

The above superelastic material may be formed of a molecular crystal body or may also be formed of a mixture that includes a molecular crystal body.

The above molecular crystal body may be a single-crystal body or may also be a polycrystal body.

The mixture which includes the above molecular crystal body may include the molecular crystal body and a matrix material.

The superelastic material may preferably have an energy storage density of 1 MJm⁻³ or less.

According to another aspect of the present invention, there are provided an energy storage material, an energy absorption material, an elastic material, an actuator, and a shape memory material that each comprise the above superelastic material.

### [Advantageous Effect of the Invention]

According to the present invention, there is provided a superelastic material that can exhibit superelasticity even with a reduced amount of energy compared with the conventional superelastic materials. There is also provided an energy storage material, an energy absorption material, an elastic material, an actuator, and a shape memory material that use the above superelastic material.

### [Brief Description of Drawings]

[FIG. 1] In FIG. 1, (a) represents the molecular structure of terephthalamide; (b) illustrates a crystal body used for analysis by X-ray diffraction and fixed with resin in a state of being bent under an applied shear force; and (c) is a set of continuous images that show the recovery motion after removal of the shear force (image-capturing interval: 1/120 sec).

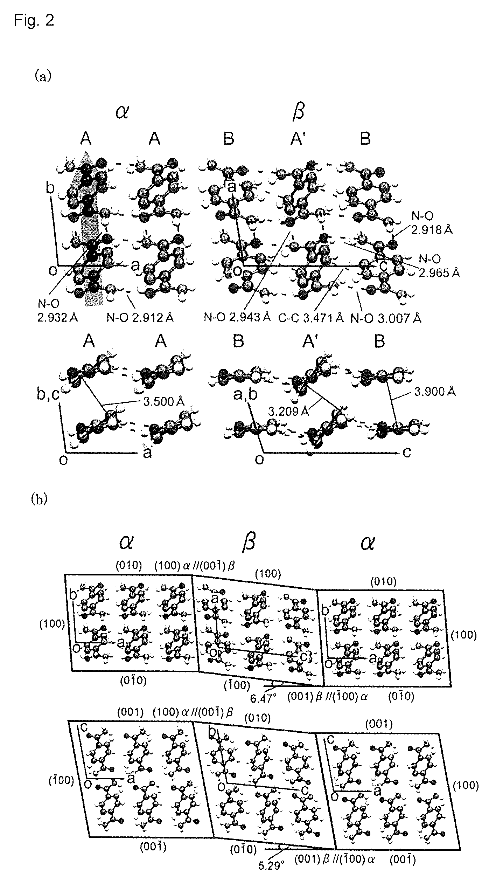

[FIG. 2] FIG. 2 is a set of diagrams for explaining phase connection under a shear-induced transition state, wherein (a) is a set of views that illustrate the crystal packing; and (b) is a set of projection images in the [001] (a) direction and the [010] (b) direction. The arrow in (a) indicates the direction of end-to-end coordination of terephthalamide arranged in the longitudinal direction. The broken lines in (a) represent the intermolecular interaction, wherein the black dotted lines each indicate a hydrogen bond between the hydrogen of an amino group and the oxygen of a carbonyl group, and the gray dotted lines each indicate the CH-π interaction between the hydrogen bound to a phenyl group and the carbon of a phenyl group. The angles in (b) are each calculated using a crossing axis of the normal vector of each crystal plane facing the boundary.
[FIG. 3] FIG. 3 is a set of images of a sample from the side surface observed using a polarization microscope (shift angle from the crossed Nicols angle: 10°), which represent the results of a shear stress test (displacement speed: 500 µm/min, 294K) for the crystal body according to Example 1, wherein the characters and numerals in the images correspond to those in FIGS. 4 and 6.
[FIG. 4] FIG. 4 is a graph that shows the temporal change in the measured force (unit: N), which represents the result of a shear stress test (displacement speed: 500 µm/min, 294K) for the crystal body according to Example 1, wherein the right-hand side vertical axis in the graph indicates a shear stress (unit: MPa) obtained by normalization with the cross-section area of the single crystal body.
[FIG. 5] FIG. 5 is a graph that shows, in a similar form to that in FIG. 4, the result of repeating 100 times the operation to vary the position of a blade which provides the graph shown in FIG. 4.
[FIG. 6] FIG. 6 is a graph that shows the relationship between the measured force (unit: N) and the displacement (unit: mm) of the blade, which represents the result of a shear stress test (displacement speed: 500 µm/min, 294K) for the crystal body according to Example 1, wherein the right-hand side vertical axis in the graph indicates a shear stress (unit: MPa) obtained by normalization with the cross-section area of the single crystal body.

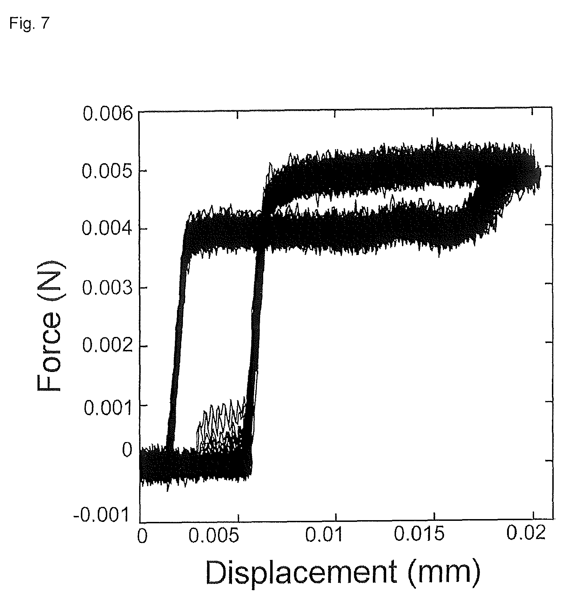

[FIG. 7] FIG. 7 is a graph that shows, in a similar form to that in FIG. 6, the result of repeating 100 times the operation to vary the position of the blade which provides the graph shown in FIG. 6.

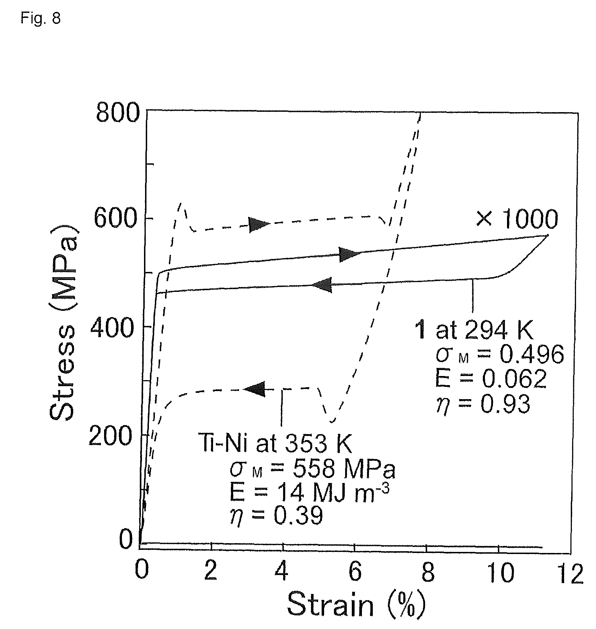

[FIG. 8] FIG. 8 is a diagram that shows an average force-displacement curve of the crystal body according to Example 1, which is obtained on the basis of the test of FIG. 7, so that the curve can be compared with a typical force-displacement curve of a Ti-Ni alloy.
[FIG. 9] FIG. 9 is a diagram that shows the simulation result of powder patterns based on the measurement results of X-ray diffraction for the β phase of the crystal body according to Example 1.
[FIG. 10] FIG. 10 represents the chemical structure of pyrazine-added copper(II) benzoate.
[FIG. 11] In FIG. 11, (a) is a schematic view that illustrates a shear stress test for the crystal body according to Example 2; and (b) is a set of observation images of the crystal body at respective positions in FIG. 13.
[FIG. 12] FIG. 12 is a set of projection images to the surface of which the normal line is the b-axis of the crystal body according to Example 2, which are for explaining phase connection under a shear-induced transition state of the crystal body according to Example 2.
[FIG. 13] FIG. 13 is a graph that shows the relationship between the measured force (unit: N) and the displacement (unit: mm) of a blade, which represents the result of a shear stress test for the crystal body according to Example 2, wherein the right-hand side vertical axis in the graph indicates a shear stress (unit: MPa) obtained by normalization with the cross-section area of the single crystal body.
[FIG. 14] FIG. 14 is a graph that shows the result of reproducing 100 times the operation to vary the position of a blade which provides the graph shown in FIG. 13, wherein the horizontal axis is the elapsed time.
[FIG. 15] FIG. 15 is a graph that shows, in a similar display form to that in FIG. 13, the result of reproducing 100 times the operation to vary the position of the blade which provides the graph shown in FIG. 13.
[FIG. 16] FIG. 16 represents the molecular structure of 3,5-difluorobenzoic acid.
[FIG. 17] In FIG. 17, (a) is a schematic view that illustrates a shear stress test for the crystal body according to Example 3; and (b) is a set of observation images of the crystal body at respective positions in FIG. 18.
[FIG. 18] FIG. 18 is a graph that shows the temporal change in the measured force (unit: N), which represents the result of a shear stress test for the crystal body according to Example 3, wherein the right-hand side vertical axis in the graph indicates a shear stress (unit: MPa) obtained by normalization with the cross-section area of the single crystal body.
[FIG. 19] FIG. 19 is a graph that shows the relationship between the measured force (unit: N) and the displacement (unit: mm) of a blade, which represents the result of a shear stress test for the crystal body according to Example 3, wherein the right-hand side vertical axis in the graph indicates a shear stress (unit: MPa) obtained by normalization with the cross-section area of the single crystal body.
[FIG. 20] FIG. 20 represents the molecular structure of tetrabutylphosphonium tetraphenylborate.
[FIG. 21] In FIG. 21, (a) is a schematic view that illustrates a shear stress test for the crystal body according to Example 4; and (b) is a set of observation images of the crystal body at respective positions in FIG. 22.
[FIG. 22] FIG. 22 is a graph that shows the temporal change in the measured force (unit: N), which represents the result of a shear stress test for the crystal body according to Example 4, wherein the right-hand side vertical axis in the graph indicates a shear stress (unit: MPa) obtained by normalization with the cross-section area of the single crystal body.
[FIG. 23] FIG. 23 is a graph that shows the temporal change in the measured force (unit: N), which represents the result of a shear stress test for the crystal body according to Example 4, wherein the effect of deformation of a stand that supports the crystal body is eliminated compared with the graph shown in FIG. 22.
[FIG. 24] FIG. 24 is a graph that shows the result of reproducing 50 times the operation to vary the position of a blade which provides the graph shown in FIG. 23, wherein the horizontal axis is the elapsed time.
[FIG. 25] FIG. 25 is a set of observation images that show the results of a shear stress test performed for the crystal body according to Example 5 while varying the ambient temperature.
[FIG. 26] FIG. 26 is a DSC chart for tetrabutylphosphonium tetraphenylborate.
[FIG. 27] FIG. 27 is a set of observation images that show the results of a shear stress test performed for the crystal body according to Example 6 while varying the ambient temperature.
[FIG. 28] FIG. 28 is an observation image of the crystal body produced in Example 8 when weights are hung from the crystal body in a room temperature environment.
[FIG. 29] FIG. 29 is a schematic diagram that illustrates the observation image of FIG. 28 so that the phase distribution of single crystal can be recognized.
[FIG. 30] FIG. 30 is an observation image of the crystal body produced in Example 8 when the atmosphere for the crystal body is maintained at 404.2K.
[FIG. 31] FIG. 31 is a schematic diagram that illustrates the observation image of FIG. 30 so that the phase distribution of single crystal can be recognized.
[FIG. 32] FIG. 32 is an observation image of the crystal body produced in Example 8 when the atmosphere for the crystal body is raised to 405K.
[FIG. 33] FIG. 33 is a schematic diagram that illustrates the observation image of FIG. 32 so that the phase distribution of single crystal can be recognized.
[FIG. 34] FIG. 34 is an observation image of the crystal body produced in Example 8 when the atmosphere for the crystal body is raised to 406.6K.
[FIG. 35] FIG. 35 is a schematic diagram that illustrates the observation image of FIG. 34 so that the phase distribution of single crystal can be recognized.
[FIG. 36] FIG. 36 is an observation image of the crystal body produced in Example 8 when the atmosphere for the crystal body is raised to 409.2K.
[FIG. 37] FIG. 37 is a schematic diagram that illustrates the observation image of FIG. 36 so that the phase distribution of single crystal can be recognized.

### [Best Mode for Carrying out the Invention]

Hereinafter, the present invention will be described in detail.

The superelastic material according to an embodiment of the present invention has a molecular crystal. In the present description, the "molecular crystal" means a crystal in which repetitive elements (unit cells) that constitute the crystal are composed of molecules, and bonds that connect the unit cells comprise bonds (e.g. Van der Waals interaction, hydrogen bond) other than covalent bond-like bonds (including metallic bonds).

Specific structure of molecules that constitute the unit cells of the molecular crystal according to an embodiment of the present invention is not particularly limited. In view of making it easy for the bonds that connect the unit cells to comprise bonds other than covalent bond-like bonds (including metallic bonds), the above molecular crystal may preferably have an organic skeleton.

That is, an example of the molecular crystal of the superelastic material according to an embodiment of the present invention may have an organic skeleton. Examples of molecules having an organic skeleton include organic molecules, organic metal molecules (metal complexes may be exemplified as specific examples) and organic-inorganic molecules, and combination thereof, which may include carbon-carbon bonds.

The superelastic material according to an embodiment of the present invention may comprise a molecular crystal body. In the present description, the "molecular crystal body" means a body that is formed of a molecular crystal. According to the feature that at least a part of the molecular crystal or crystals included in the molecular crystal body of the superelastic material of an embodiment of the present invention can exhibit superelasticity, the superelastic material of an embodiment of the present invention can exhibit superelasticity as the whole material. The molecular crystal body of the superelastic material according to an embodiment of the present invention may be a single-crystal body or may also be a polycrystal body. In the present description, the concept of the term "single-crystal body" encompasses a mosaic crystal and a crystal that includes lattice defects as well as a crystal of which the degree of crystallization is low and a crystal of which the degree of purity is low. In the present description, the term "polycrystal body" means an aggregate of crystals in contrast to a single crystal.

A specific example of the superelastic material according to an embodiment of the present invention may be formed of a molecular crystal body. Another specific example of the superelastic material according to an embodiment of the present invention may be formed of a mixture that includes a molecular crystal body. The content ratio of the molecular crystal body in the above mixture is not particularly limited, provided that the mixture as a whole can exhibit superelasticity. Specific example of the mixture may be a case in which the mixture includes a molecular crystal body and a matrix material. In this case, the mixture may have a structure in which the molecular crystal body is dispersed in the matrix material, for example, so that the mechanical characteristics of the molecular crystal body dominate the mechanical characteristics of the mixture as a whole, and the mixture as a whole appears to be able to exhibit superelasticity. Here, the relation between the matrix material and molecular crystal body of the mixture is not limited. The matrix material may have a larger volume than that of the molecular crystal body, a comparable volume to that of the molecular crystal body, or a smaller volume than that of the molecular crystal body. The degree of interaction between the matrix material and molecular crystal body of the mixture is also not limited. The matrix material and the molecular crystal body may be unified, such as that the substance which constitutes the matrix material is associated with or chemically bound to the substance which constitutes the molecular crystal body. Such an example may be a case in which the mixture comprises a crosslinked structure of the matrix material and molecular crystal body. Another example may be a case in which the mixture comprises a high-molecular substance, and partial structures (e.g. side chains) of the high-molecular substance are spatially arranged in a regular manner, such as due to aggregation. In this case, the partial structures arranged in a regular manner belong to the molecular crystal body, and portions that are not arranged in a regular manner in the high-molecular substance belong to the matrix material.

The superelastic material according to an embodiment of the present invention may involve a loading transformation process and an unloading reverse transformation process. In the present description, the "loading transformation process" means a process in which, when an external force is applied to the superelastic material according to an embodiment of the present invention, the applied external force triggers transformation and progresses the transformation. In the present description, the "unloading reverse transformation process" means a process in which reducing the external force applied to the superelastic material in the state where the loading transformation process progresses triggers reverse transformation, and the reduced external force progresses the reverse transformation.

Temperature range within which the reverse transformation readily occurs in the superelastic material according to the present embodiment is not particularly limited. When thermoelastic martensitic transformation occurs in the superelastic material according to the present embodiment, the stress-induced phase may be destabilized within a temperature region of which the lower limit value is equal to the reverse transformation finishing temperature, so that the reverse transformation is likely to occur. In contrast, the stress-induced phase can stably exist within a temperature region of the reverse transformation starting temperature or lower, so that the reverse transformation is less likely to occur. Therefore, when the thermoelastic martensitic transformation occurs in the superelastic material and an external force is applied thereto within a temperature region of the reverse transformation starting temperature or lower, the strain caused by the external force may remain in the superelastic material. Thereafter, if the material in which the strain remains is heated to a temperature of the reverse transformation finishing temperature or higher, the reverse transformation may recover the strain. Thus, when the thermoelastic martensitic transformation occurs in the superelastic material according to the present embodiment, the shape-memory effect of the superelastic material can be observed. In the superelastic material having a molecular crystal according to the present embodiment, the molecules that constitute the unit cells may be appropriately selected such that the reverse transformation can readily occur at the room temperature (23°C) or otherwise cannot readily occur at the room temperature.

The degree of superelasticity possessed by the superelastic material can be evaluated in accordance with some parameters.

An example of such parameters may be an energy storage density. The energy storage density refers to a value (unit: Jm⁻³) obtained by dividing work, which is done by the superelastic material when the amount of displacement is reduced and which can be obtained from a force-displacement curve, by a volume before transformation of a transformed portion of the superelastic material in a state in which the amount of displacement is maximum. The energy storage density in a Ti-Ni alloy, which is a typical example of the conventional metal-based superelastic material, may be 14 MJm⁻³, i. e. 10 MJm⁻³ order. In contrast, the energy storage density of the superelastic material according to an embodiment of the present invention may be 1 MJm⁻³ or less, the energy storage density of a preferred example of the superelastic material according to an embodiment of the present invention may be 0.2 MJm⁻³ or less, the energy storage density of another preferred example of the superelastic material according to an embodiment of the present invention may be 0.1 MJm⁻³ or less, and the energy storage density of still another preferred example of the superelastic material according to an embodiment of the present invention may be 50 kJm⁻³ or less.

Another example of the above parameters may be a chemical stress. The chemical stress refers to an average value (unit: Pa) of a stress calculated from an external force, which is applied in a state where the variation in numerical value of the external force apparently becomes small as the transformation progresses and which can be obtained from a force-displacement curve in the loading transformation process, and a recovery force which is generated in a state where the variation in numerical value of the recovery force apparently becomes small as the reverse transformation progresses and which can be obtained from a force-displacement curve in the unloading reverse transformation process. The chemical stress in a Ti-Ni alloy, which is a typical example of the conventional metal-based superelastic material, may be 558 MPa, i.e. the order of 100 MPa to 1 GPa. In contrast, the chemical stress of the superelastic material according to an embodiment of the present invention may be 10 MPa or less, and the chemical stress of a preferred example of the superelastic material according to an embodiment of the present invention may be 1 MPa or less. As the chemical stress decreases, the external force required for exhibiting the superelasticity tends to become low, and the superelastic material can readily be applied to microstructures.

Still another example of the above parameters may be a superelastic index. The superelastic index refers to a value (unit: dimensionless) obtained by dividing the energy storage density by the chemical stress. The superelastic index of a Ti-Ni alloy, which is a typical example of the conventional metal-based superelastic material, may be about 0.025, i.e. less than 0.05. In contrast, the superelastic index of the superelastic material according to an embodiment of the present invention may be 0.05 or more, and the superelastic index of a preferred example of the superelastic material according to an embodiment of the present invention may be 0.1 or more. It can be said that, the higher the superelastic index is, the more efficient the superelastic material is, i.e., the superelastic material can store a large amount of energy even with a small external force applied. Therefore, the superelastic material according to an embodiment of the present invention, which has a higher superelastic index than that of the conventional superelastic material, can be expected to be readily applied to microstructures.

In the superelastic material according to an embodiment of the present invention, the transformation can occur to have a large displacement with a small input of energy. In other words, it can be said that a small output can be uniformly achieved with large reverse transformation. Therefore, the following uses are expected:
an energy storage material that comprises the superelastic material according to an embodiment of the present invention;
an energy absorption material that comprises the superelastic material according to an embodiment of the present invention;
an elastic material that comprises the superelastic material according to an embodiment of the present invention;
an actuator that comprises the superelastic material according to an embodiment of the present invention; and
a shape memory material that comprises the superelastic material according to an embodiment of the present invention.

### [Examples]

Hereinafter, effects of the present invention will be described with reference to examples, but the present invention is not limited to these examples.

### (Example 1)

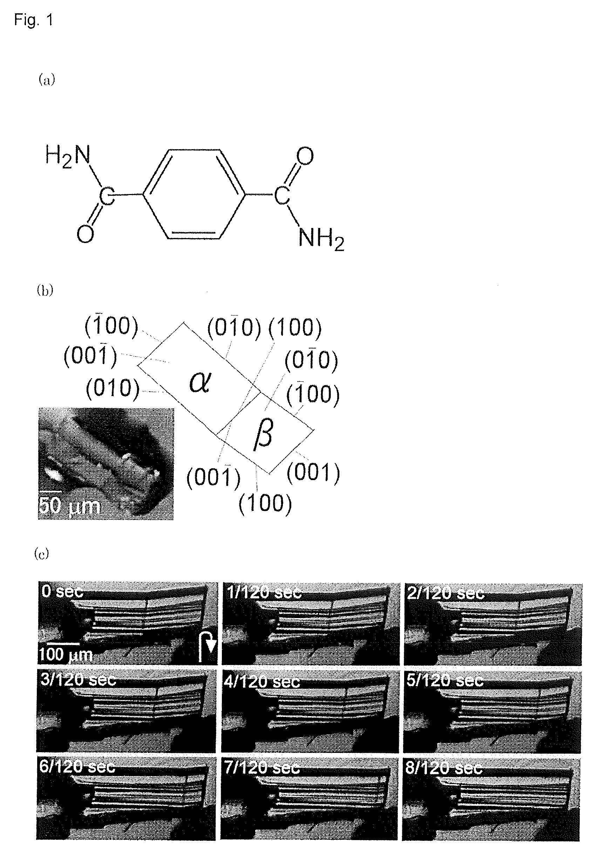

A well-formed single crystal of terephthalamide was obtained by recrystallization of reagent-grade terephthalamide from warm water. A crystal body obtained by drying the single crystal in a vacuum was used in the test. When a shear force (referred to as "shear force 1," hereinafter) was applied on the {010} crystal surface of the crystal body, the crystal body was bent and another crystal phase was generated therein. The boundary between the original crystal phase and the generated other crystal phase was sharp. When the crystal body was continued to be pushed, i.e., when the shear force 1 was continued to be applied, the bending position at the phase boundary in the crystal body transferred as shown in FIG. 1.

When applying the shear force 1 was removed, the bent crystal body spontaneously restored, and the phase boundary, which moved due to applying the shear force 1, transferred back with a speed of 3.33 mm/sec at 294K.

X-ray diffraction measurement was performed for the crystal body in a state of being applied with the shear force 1. From the results, the shear force-induced transition from a mother phase (α-phase) to a daughter phase (β-phase) was observed (FIG. 1) . While both of the α-phase and the β-phase had the same P-1-type crystal system, the crystal packing was significantly different. The crystal structure of the α-phase was a structure that was already known, the structure of the β-phase was a structure that was not known.

In the α-phase, a plurality of terephthalamide molecules gathered to form polymeric sheets. In the sheets, the terephthalamide molecules constituted a network structure in which the terephthalamide molecules were bound by hydrogen bonds between NH portions and O=C portions. The distance between N and O of the hydrogen bond formed of NH and O=C of the ends along the longitudinal axis of terephthalamide was 2.932(4) Å, and the distance between N and O of the hydrogen bond formed of NH and O=C of ad j a cent terephthalamide molecules, i.e. molecules located at positions in the direction perpendicular to the longitudinal axis in the sheets, was 2.912(3) Å.

In the change from the α-phase to the β-phase due to applying the shear force 1, the sheets formed of a uniform -A-A-A-A- arrangement transformed into sheets formed of an alternative -A'-B-A'-B- arrangement. As illustrated in FIG. 2, the β-phase had a structure in which columns A' substantially maintaining the structure of A were sandwiched by the deformed columns B.

The change in the molecular arrangement from the α-phase into the β-phase achieved a relatively denser packing state. Specifically, the density of the α-phase was 1.470 Mg/m⁻³, and the density of the β-phase was 1.484 Mg/m⁻³. In the β-phase, the terephthalamide molecules also constituted a network structure in which the terephthalamide molecules were bound by hydrogen bonds between NH and O=C. The change in the hydrogen bond distance was slightly observed. The distance between N and O of the hydrogen bond formed of NH and O=C of the ends along the longitudinal axis of terephthalamide was 2.943 (5) Å in the column A' and 2.918 (5) Å in the column B. With regard to the hydrogen bond formed of NH and O=C of terephthalamide molecules located at positions in the direction perpendicular to the longitudinal axis in the sheets, the distance between N and O of the hydrogen bond formed of NH in the column A' and O=C in the column B was 3.007 (4) Å, and the distance between N and O of the hydrogen bond formed of NH in the column B and O=C in the column A' was 2.965(4) Å.

As illustrated in FIG. 2, additional interaction was recognized in the β-phase. In the sheets, CH-n interaction was recognized, which was specifically the interaction between H bound to a phenyl group in the column B and C of a phenyl group in the column A'. The distance between C of the phenyl group to which H was bound in the column B and C of the phenyl group in the column A' was 3.471 (6) Å.The manner of layer stack of the phenyl groups changed between in the α-phase and in the β-phase. In the α-phase, the phenyl groups were weakly stacked in the column A in which the surface separation of the phenyl groups was 3,500 Å, resulting in a stack structure having high two-dimensionality. Whereas in the β-phase, the surface separation of the phenyl groups was significantly shortened to 3,209 Å in the column A', and an intermolecular interaction network was formed three-dimensionally.

The heterophase boundary (martensite habit plane) parallel with the (100) plane of the α-phase was almost perpendicular to the (011) plane as the cleavage plane of the α-phase. The (011) plane of the α-phase was a plane that lay in the sheets constituted of hydrogen bonds.

The disparity between the habit plane and the cleavage plane allowed the transformation stable against the distortion around the heterophase interface.

From the data of X-ray diffraction for the crystal body in the bending form, it was estimated how each crystal fitted at the interface under the α-phase/β-phase coexistent state. In the interface between the (100) plane of the α-phase and the (00-1) plane of the β-phase, the ratio of facing area (S_{α}/S_{β}) was 0.989, which raises the Bain distortion. It appeared that, at the interface, the crystal face of the α-phase and the crystal face of the β-phase respectively received face contraction and extension. In detail, a certain difference was recognized in the length of c-axis of the α-phase (7.1853 Å) and b-axis of the β-phase (7.268 Å), and the difference indicates the mainly unidirectional distortion along the c-axis in the bc face and a unidirectional distortion along the b-axis in the ab face, respectively.

The deduced connection views at the interfaces (FIG. 2) revealed that the martensitic transformation occurred in the observed twinning transformation in the crystal body. The views also showed the possibility that the [010] direction and [00-1] direction in the α-phase is effective for shear-induced twinning transformation.

The bending angle of the crystal body observed using a microscope was within a range of 5° to 8° judged from the projection direction of [001] and within a range of 3° to 5° judged from the projection direction of [010]. These angles agree approximately with the angles expected from the X-ray diffraction data (6.55° and 5.35°) (FIG. 2).

The proportion in the displacements, obtained by the microscope observation, of the shear force 1 applying position onto the (010) surface of the α-phase and the moving position (farther from the position applied with the shear force 1) of the β-phase/α-phase interface was 1:6.7. This proportion agrees approximately with the expected proportion (1:5.4) using the X-ray diffraction data.

The agreement to these parameters demonstrates the harmonious support of the microscopic structural change and the macroscopic structural change in the crystal body according to the present example.

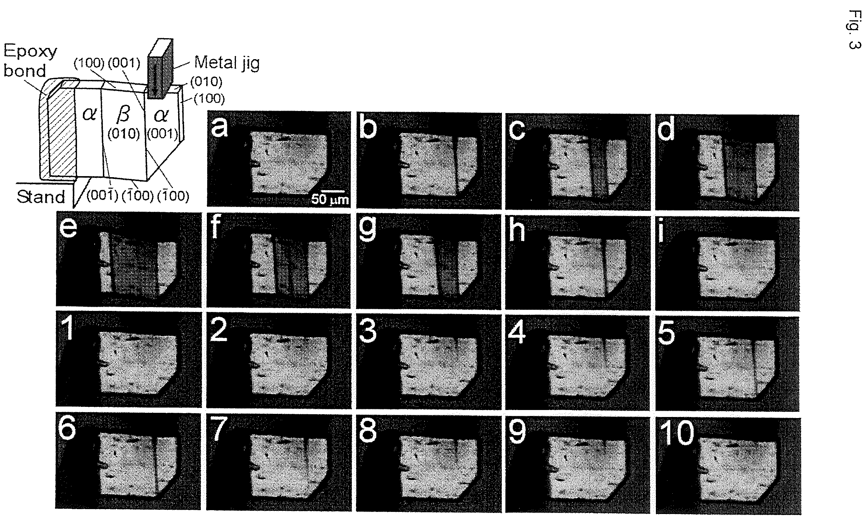

The relationship between the shear force and the deformation was investigated by applying a load to a part of the {010} crystal surface. The load in this direction appears to be nearly parallel to the shear-transition easy axis (see FIG. 2). One end portion of a crystal body (thickness of 149.52 µm and width of 58.84 µm) formed of the above single crystal of terephthalamide was fixed, and a metallic blade of a width of 25 µm was made to come into contact with the (010) surface of the crystal body and pushed across the surface at a constant speed of 500 µm/min at 294K. This resulted in the twinning transformation of the crystal body as shown in FIG. 3.

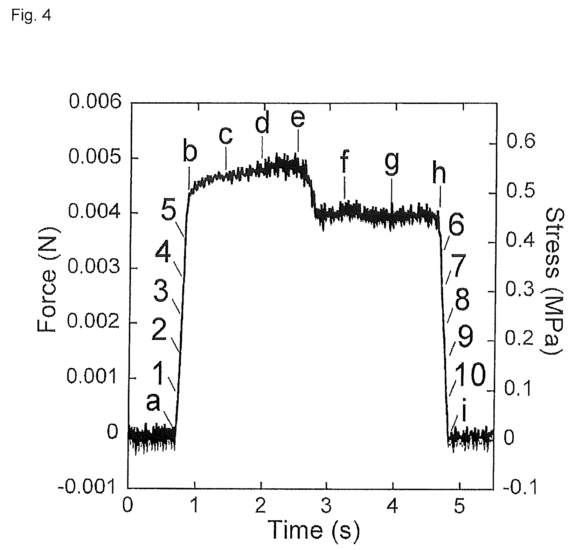

As shown in FIG. 4, stress was detected at the position of (a) after the blade reached the crystal body. Thereafter, the phase boundary began to increase as the load increased (images denoted by numerals 1 to 5 in FIG. 3). The stress became constant at (b) of FIG. 4 where a thin band of β-phase was generated from the contact portion of the blade.

Subsequently, as shown in (c) and (d) of FIG. 4, the state of a stable stress value continued during the growth and propagation of the β-phase by continuous proceeding of the α-phase/β-phase transition.
(e) of FIG. 4 is the switching point for the unloading process (pulling back the blade), and a slight decrease in the stress was observed. Thereafter, the state of a stable stress value continued during the phase change of the β-phase into the α-phase. At (h) of FIG. 4, the region of β-phase narrowed to be a boundary line. From this point, the stress was linearly decreased until the blade was removed from the surface of the crystal body at (i) of FIG. 4, and during this period, a phenomenon occurred that the line-like boundary decreased and vanished (images denoted by numerals 6 to 10 in FIG. 3).

The shear stress curve thus obtained (FIG. 4) clearly demonstrated that the crystal body according to the present example exhibited superelasticity.

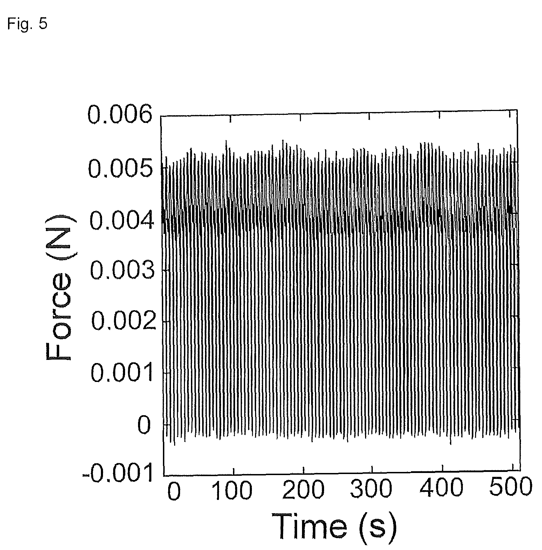

As shown in FIG. 5, the above superelastic transformation in the crystal body according to the present example was reproduced in 100 cycles of measurement without any elastic reduction or material deterioration.

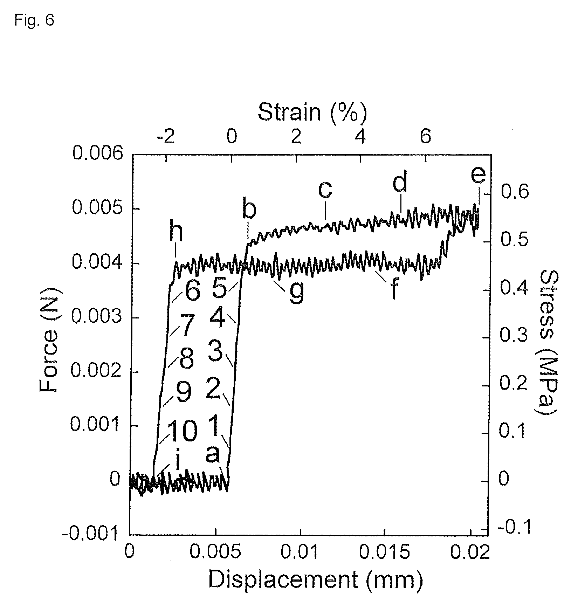

As a result of the cyclic test on crystal bodies formed of single crystals with different dimensions, the strength of stress generated under the bending state of the crystal bodies was linear with the surface area of the phase boundary, and was not correlated with the contact surface area with the blade. This result means that the elastic index as for shear should be normalized by a parameter that is obtained by dividing the absolute force by the surface area of the α-phase/β-phase boundary. The above parameter corresponds to a shear stress that is obtained by dividing the external force by the cross-sectional area of the crystal body. From FIG. 4, the shear stress in the transformation (from the α-phase to the β-phase) of the superelastic material formed of the crystal body according to the present example was 0.496 MPa, and the shear stress in the reverse transformation (from the β-phase to the α-phase) was 0.459 MPa. This shear stress was about one thousand times smaller than 558 MPa, which is known as the shear stress of a typical Ti-Ni alloy. The chemical stress, which is the average value of the shear stress at the position of (b) and the shear stress at the position of (h) in FIG. 6, was 0.477 MPa.

The energy storage density and energy storage efficiency (η) of the superelastic material formed of the crystal body according to the present example were estimated to be 0.062 MJm⁻³ and 0.925, respectively. The superelastic index of the superelastic material formed of the crystal body according to the present example was estimated to be about 0.13.

The movable range of superelastic shear strain was wide, and the ratio of the displacement amount to the crystal size was up to 11.34%. This numerical value can be predicted by the geometric arrangement of the junction of adjacent crystal lattices with the X-ray diffraction data.

The superelastic material formed of a single crystal body of terephthalamide according to the present example is a pure organic crystal body, i.e. an organic crystal body that does not have an inorganic component such as metal elements. As described above, its energy storage density is 0.062 MJm⁻³, which is 226 times less than the energy storage density of a typical Ti-Ni alloy (14 MJm⁻³). When the above energy storage density is converted to an amount per mol, the energy storage density of the superelastic material formed of a single crystal body of terephthalamide is 6.96 Jmol⁻¹ while the energy storage density of a typical Ti-Ni alloy is 114.76 Jmol⁻¹, and the ratio thereof is 1:16.5. This ratio agrees approximately with 1:15.9 which is the ratio in the bonding energy of the superelastic material formed of a single crystal body of terephthalamide (4 times the bonding energy between NH and O=C = 28.87 kJmol⁻¹×4) to the bonding energy of Ti-Ni alloy (458.83 kJmol⁻¹×4). Thus, the energy storage ability is related to the lattice energy. The volumetric energy density may become relatively smaller by the extent of the lattice. Consequently, the superelastic molecular material provided according to the present example can produce a transformation with a large displacement from a small energy input. In other words, it can be said that the superelastic molecular material can generate a uniform level of small power with a large reverse transformation.

Table 1 shows the measurement results of X-ray diffraction for the β-phase of the superelastic material formed of a crystal body of terephthalamide according to the present example. The measurement was performed using an X-ray diffractometer, Smart APEX CCD, available from Bruker Corporation.

**[Table 1]**

| | |
|---|---|
| Empirical formula | C8 H8 N2 O2 |
| Formula weight | 164.16 |
| Temperature | 298 K |
| Wavelength | 0.71073 Å |
| Crystal system | Triclinic |
| Space group | P-1 |
| Unit cell dimensions | a = 5.3663(17) Å a= 104.149(7)°. |
| | b = 7.268(2) Å b= 97.699(7)°. |
| | c = 10.160(3) Å g = 102.672(7)°. |
| Volume | 367.4(2) Å³ |
| Z | 2 |
| Density (calculated) | 1.484 Mg/m³ |
| Absorption coefficient | 0.110 mm⁻¹ |
| F(000) | 172 |
| Crystal size | 0.13x0.11 x0.04mm³ |
| Theta range for data collection | 2.11 to 25.01°. |
| Index ranges | -6<=h<=6, -7<=k<=8, -12<=l<=11 |
| Reflections collected | 2118 |
| Independent reflections | 1295 [R(int) = 0.0284] |
| Completeness to theta = 25,01° | 99,50% |
| Absorption correction | Empirical |
| Max. and min. transmission | 0.9956 and 0.9859 |
| Refinement method | Full-matrix least-squares on F² |
| Data / restraints / parameters | 1295/0/109 |
| Goodness-of-fit on F² | 1.125 |
| Final R indices [l>2sigma(l)] | R1 = 0.0868, wR2 = 0.2292 |
| R indices (all data) | R1 = 0.1045, wR2 = 0.2468 |
| Largest diff. peak and hole | 0.550 and -0.412 e.Å⁻³ |

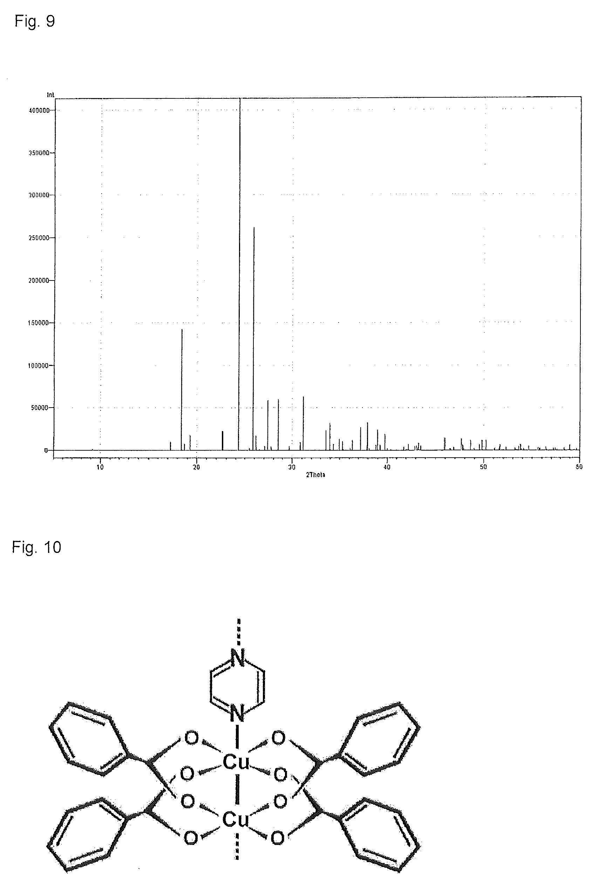

FIG. 9 shows the simulation result of powder patterns performed on the basis of the above data.

### (Example 2)

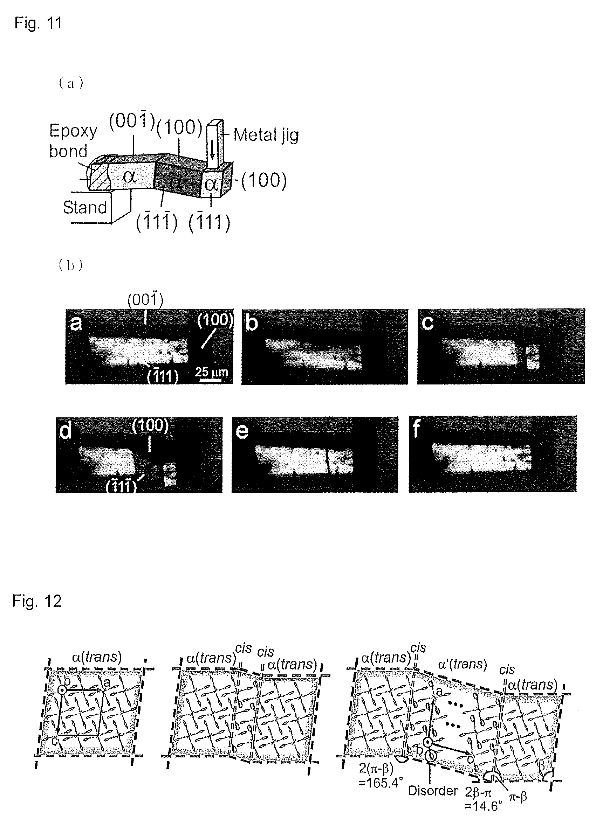

A single crystal of pyrazine-added copper(II) benzoate having a chemical structure shown in FIG. 10 was produced using the method as described in Patent No. 4951728. A crystal body obtained by drying the single crystal in a vacuum was used in the test. When a shear force (referred to as "shear force 2," hereinafter) was applied on the {00-1} crystal surface of the crystal body, the crystal body was bent and another crystal phase was generated therein. The boundary between the original crystal phase and the generated other crystal phase was sharp. When the crystal body was continued to be pushed, i.e., when the shear force 2 was continued to be applied, the bending position at the phase boundary in the crystal body transferred as shown in FIG. 11. When applying the shear force 2 was removed, the bent crystal body spontaneously restored, and the phase boundary, which moved due to applying the shear force 2, proceeded in the opposite direction.

X-ray diffraction measurement was performed for the crystal body in a state of being applied with the shear force 2. From the results, the shear force-induced transition from a mother phase (α(trans)-phase) to a daughter phase (α'(trans)-phase) was observed (FIG. 12). The bending angle expected from the X-ray diffraction data was 14.6°.

The relationship between the shear force and the deformation was investigated by applying a load to a part of the {00-1} crystal surface. One end portion of a crystal body formed of the above single crystal of pyrazine-added copper (II) benzoate was fixed, and a metallic blade of a width of 25 µm was made to come into contact with the (00-1) surface of the crystal body and pushed across the surface at a constant speed of 500 µm/min at 294K.

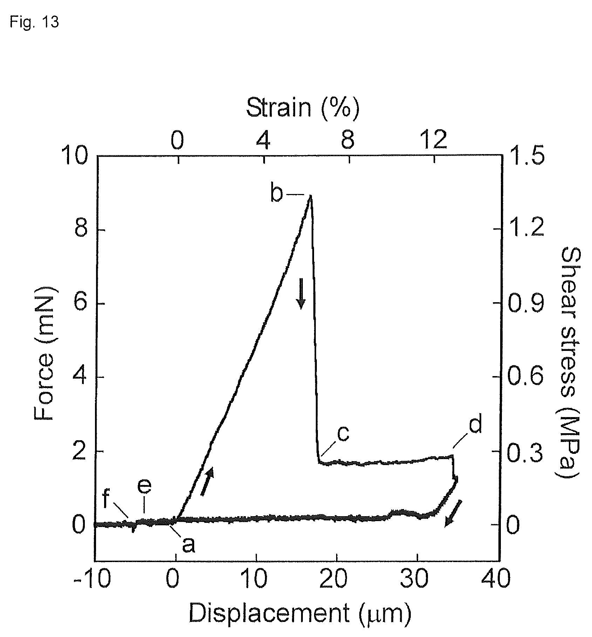

As a result, the stress was detected at the position of (a) as shown in FIG. 13 after the blade reached the crystal body. Thereafter, the stress increased substantially in proportion to the displacement amount of the blade. Phase transition did not occur in this process, but the daughter phase was generated due to phase transition at the position of (b), and decrease in the stress was measured until the position (c) where the displacement amount of the blade slightly increased. This resulted in a structure in which a mother phase, daughter phase and mother phase were arranged in this order. The plane of the daughter phase interposed between the (00-1) planes of the two mother phases was (100) plane. When the displacement amount of the blade was further increased from the position of (c), the stress increased very little, and the daughter phase grew (i.e., transition from the mother phase to the daughter phase progressed).
(d) in FIG. 13 is the switching point for the unloading process (pulling back the blade), and a slight decrease in the stress was observed. Thereafter, the state of a stable stress value continued, during the phase change of the daughter phase into the mother phase. At (e) of FIG. 13, the region of daughter phase narrowed to be a boundary line between two mother phases. The line-like boundary then vanished at (f) of FIG. 13.

The shear stress curve thus obtained (FIG. 13) clearly demonstrated that the crystal body according to the present example exhibited superelasticity.

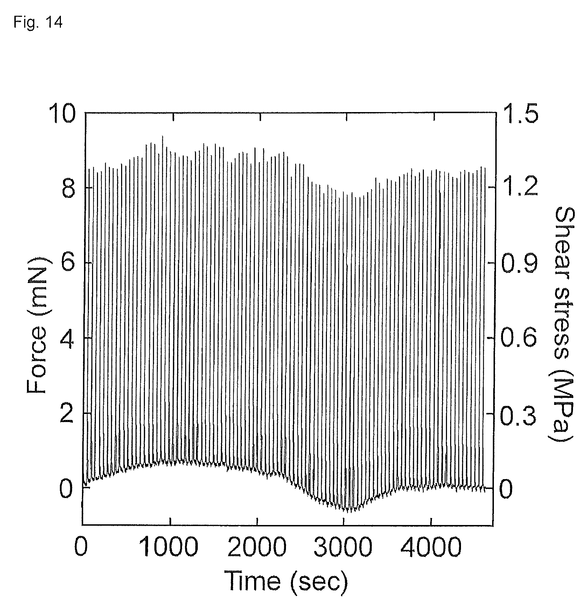

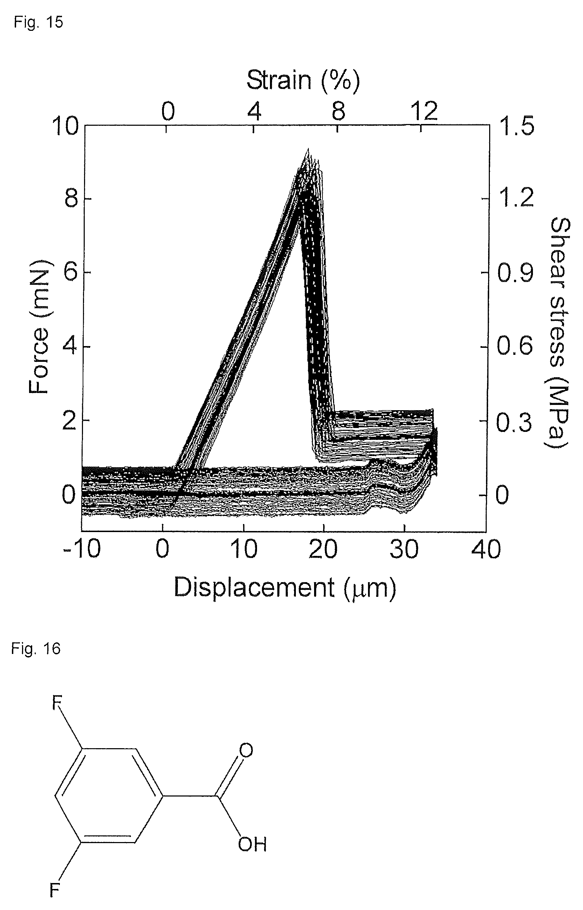

As shown in FIGS. 14 and 15, the above superelastic transformation in the crystal body according to the present example was reproduced in 100 cycles without any elastic reduction or material deterioration.

The energy storage density and energy storage efficiency (η) of the superelastic material formed of the crystal body according to the present example were estimated to be 0.0404 MJm⁻³ and 0.0539, respectively. The chemical stress, which is the average value of the shear stress at the position of (c) and the shear stress at the position of (e) in FIG. 13, was 0.146 MPa. The superelastic index of the superelastic material formed of the crystal body according to the present example was estimated to be about 0.277.

### (Example 3)

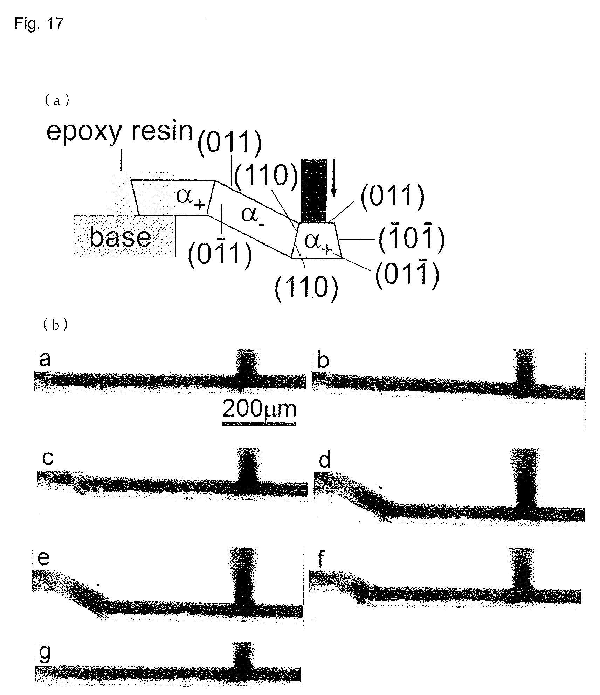

The 3,5-difluorobenzoic acid (FIG. 16) was recrystallized from warm water, and a well-formed single crystal of 3,5-difluorobenzoic acid was thereby obtained. A crystal body obtained by drying the single crystal in a vacuum was used in the test. When a shear force (referred to as "shear force 3," hereinafter) was applied on the {011} crystal surface of the crystal body, the crystal body was bent and another crystal phase was generated therein. The boundary between the original crystal phase and the generated other crystal phase was sharp. When the crystal body was continued to be pushed, i.e., when the shear force 3 was continued to be applied, the bending position at the phase boundary in the crystal body transferred as shown in FIG. 17(b). When applying the shear force 3 was removed, the bent crystal body spontaneously restored, and the phase boundary, which moved due to applying the shear force 3, proceeded in the opposite direction.

X-ray diffraction measurement was performed for the crystal body in a state of being applied with the shear force 3. The results are listed in Table 2. The measurement was performed using an X-ray diffractometer, Smart APEX CCD, available from Bruker Corporation.

**[Table 2]**

| | | | |
|---|---|---|---|
| Empirical formula | C₇H₄F₂O₂ | C₇H₄F₂O₂ | C₇H₄F₂O₂ |
| Morphology | Straight | Bent(a₊/a₋) | |
| Crystal phase | a | a₊ | a₋ |
| Empirical formula | C₇H₄F₂O₂ | C₇H₄F₂O₂ | C₇H₄F₂O₂ |
| Crystal size /mm³ | 0.78x0.12x0.10 | 0.19×0.19×0.06 | 0.20x0.19x0.06 |
| M | 158.1 | 158.1 | 158.1 |
| Crystal system | Monoclinic | Monoclinic | Monoclinic |
| Space group | *P*2₁/c | *P*2₁/c | *P*2₁/c |
| T/K | 298 | 298 | 298 |
| a/Å | 3.7616(15) | 3.772(3) | 3.7717(18) |
| b/Å | 13.365(5) | 13.396(9) | 13.385(7) |
| c/Å | 14.003(6) | 14.040(10) | 14.005(7) |
| a /deg | 90 | 90 | 90 |
| b /deg | 93.749(9) | 93.776(14) | 93.884(11) |
| g /deg | 90 | 90 | 90 |
| V/Å³ | 702.5(5) | 707.9(9) | 705.4(6) |
| Z | 4 | 4 | 4 |
| D_{calcd} / Mg m⁻³ | 1.495 | 1.483 | 1.489 |
| m(Mo Ka) / mm⁻¹ | 0.143 | 0.142 | 0.142 |
| Reflections collected | 3986 | 3839 | 5010 |
| Independent reflections (Rᵢₙₜ) | 1250(0.1004) | 1254(0.0824) | 1733(0.0714) |
| Goodness of fit | 1.051 | 0.96 | 0.937 |
| R₁(I > 2s (all data)) | 0.0856(0.1974) | 0.0776(0.1692) | 0.0934(0.2166) |
| wR₂(I > 2s (all data)) | 0.2242(0.2694) | 0.1773(0.2381) | 0.2196(0.3075) |
| Largest diff. peak (hole) /eÅ³ | 0.215(-0.259) | 0.250(-0.313) | 0.509(-0.390) |

The shear force-induced twinning transition from a mother domain (α₊-phase) to a daughter domain (α₋-phase) was observed (FIG. 17(a)).

The relationship between the shear force and the deformation was investigated by applying a load to a part of the {011} crystal surface. One end portion of a crystal body formed of the above single crystal of 3,5-difluorobenzoic acid was fixed, and a metallic blade of a width of 25 µm was made to come into contact with the (011) surface of the crystal body and pushed across the surface at a constant speed of 500 µm/min at 294K.

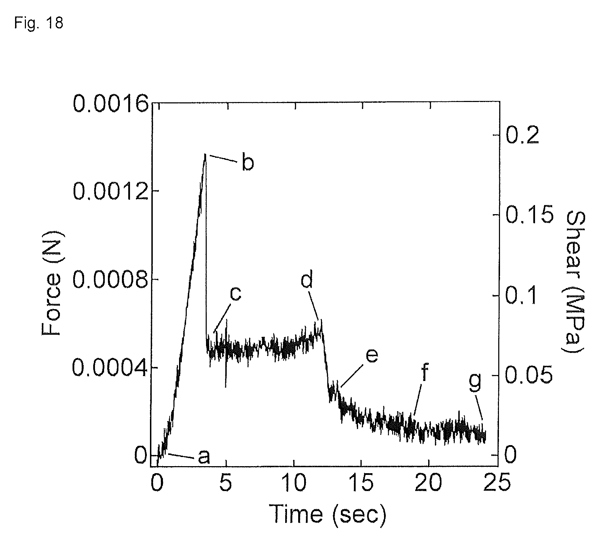

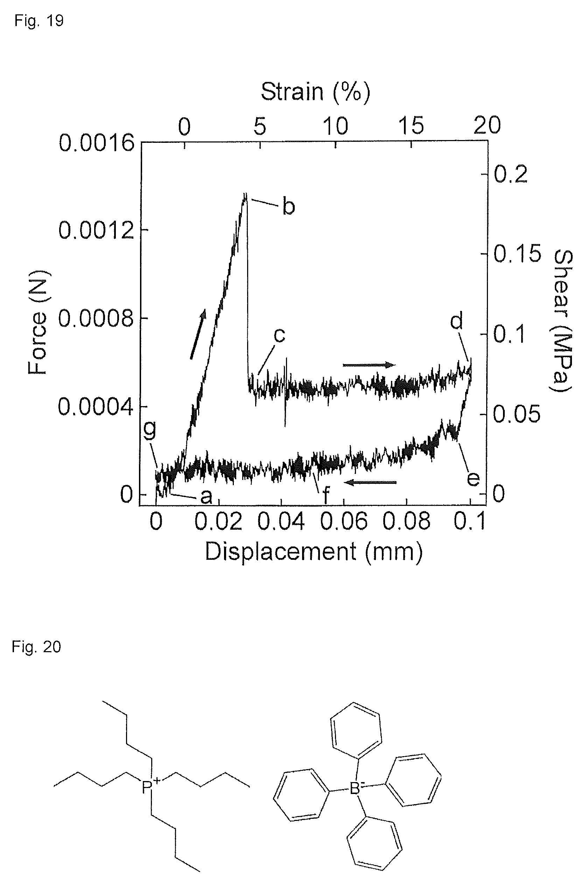

As a result, the stress was detected at the position of (a) as shown in FIGS. 18 and 19 after the blade reached the crystal body. Thereafter, the stress increased substantially in proportion to the displacement amount of the blade. Twinning transformation did not occur in this process, but the daughter domain was generated due to twinning transformation at the position of (b), and decrease in the stress was measured until the position (c) where the displacement amount of the blade slightly increased. This resulted in a structure in which a mother domain, daughter domain and mother domain were arranged in this order. When the displacement amount of the blade was further increased from the position of (c), the stress increased very little, and the daughter domain grew (i. e. , transition from the mother domain to the daughter domain progressed). The bending angle estimated from the X-ray diffraction data was 27.8°.
(d) in FIGS. 18 and 19 is the switching point for the unloading process (pulling back the blade), and a considerable amount of decrease in the stress was observed to the position of (e) after the unloading. Thereafter, the stress moderately decreased (position (f)), and during this period, the twinning generation from the daughter domain into the mother domain occurred. The daughter domain then vanished at the position (g).

The shear stress curve thus obtained (FIG. 19) clearly demonstrated that the crystal body according to the present example exhibited superelasticity.

The energy storage density and energy storage efficiency (η) of the superelastic material formed of the crystal body according to the present example were estimated to be 0.0119 MJm⁻³ and 0.178, respectively. The chemical stress, which is the average value of the shear stress at the position of (c) and the shear stress at the position of (g) in FIG. 19, was 0.0453 MPa. The superelastic index of the superelastic material formed of the crystal body according to the present example was estimated to be about 0.263.

### (Example 4)

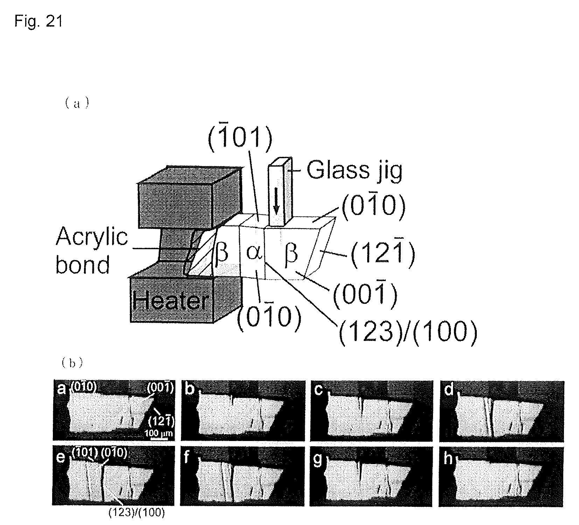

The tetrabutylphosphonium tetraphenylborate (FIG. 20, denoted as "(PⁿBu₄) (BPh₄)," hereinafter) was recrystallized from warm water, and a good single crystal of (PⁿBu₄) (BPh₄) was thereby obtained. A crystal body obtained by drying the single crystal in a vacuum was used in the test. When a shear force (referred to as "shear force 4," hereinafter) was applied on the {0-10} crystal surface of the crystal body in an environment of 400K, the crystal body was bent and another crystal phase was generated therein. The boundary between the original crystal phase and the generated other crystal phase was sharp. When the crystal body was continued to be pushed, i.e., when the shear force 4 was continued to be applied, the bending position at the phase boundary in the crystal body transferred as shown in FIG. 21(b). When applying the shear force 4 was removed, the bent crystal body spontaneously restored, and the phase boundary, which moved due to applying the shear force 4, proceeded in the opposite direction.

X-ray diffraction measurement was performed for the crystal body in a state of being applied with the shear force 4. The results are listed in Table 3. The measurement was performed using an X-ray diffractometer, Smart APEX CCD, available from Bruker Corporation.

**[Table 3]**

| | | | |
|---|---|---|---|
| Empirical formula | C₄₀H₅₆BP | C₄₀H₅₆BP | C₄₀H₅₆BP |
| Phase | α | α | β |
| Temperature /K | 298 | 394 | 403 |
| Crystal size /mm³ | 0.75x0.16x0.09 | 0.46x0.14x0.11 | 1.00x0.24x0.23 |
| M | 578.63 | 578.63 | 578.63 |
| Crystal system | Triclinic | Triclinic | Monoclinic |
| Space group | *P*-1 | *P*-1 | *P*2₁/n |
| a/Å | 10.311(3) | 10.5582(11) | 12.1401(14) |
| b/Å | 18.895(5) | 19.131(2) | 36.975(4) |
| c /A | 20.770(6) | 20.915(2) | 17.688(2) |
| a /° | 69.618(7) | 68.892(2) | 90 |
| β/° | 85.946(7) | 85.501(3) | 102.611(2) |
| γ/° | 76.898(6) | 77.175(2) | 90 |
| V/Å³ | 3694.1(18) | 3842.7(7) | 7748.1(15) |
| Z | 4 | 4 | 8 |
| D_{calcd}/Mgm³ | 1.04 | 1 | 0.992 |
| µ(Mo Kα)mm⁻¹ | 0.099 | 0.095 | 0.094 |
| Reflections collected | 21495 | 22471 | 57189 |
| Independent reflections (*R*ᵢₙₜ) | 12972 (0.0459) | 13522 (0.0410) | 19194 (0.0892) |
| Goodness of fit | 1.12 | 0.894 | 1.123 |
| *R*₁(*I* > 2σ (all data)) | 0.1120 (0.2319) | 0.0960(0.2774) | 0.1668 (0.4430) |
| _{w}*R*₂(*I* > 2σ (all data)) | 0.3496 (0.4207) | 0.2660 (0.3982) | 0.4463 (0.5654) |
| Leastdiff.peak (hole) /eÅ³ | 0.443 (-0.336) | 0.421 (-0.264) | 0.476 (-0.265) |

The shear force-induced transition from a mother phase (high-temperature phase β) to a daughter phase (low-temperature phase α) was observed (FIG. 21(a)).

The relationship between the shear force and the deformation was investigated by applying a load to a part of the {0-10} crystal surface. One end portion of a crystal body formed of the above single crystal of (PⁿBu₄) (BPh₄) was fixed, and a metallic blade of a width of 25 µm was made to come into contact with the (0-10) surface of the crystal body and pushed across the surface at a constant speed of 500 µm/min at 400K.

As a result, the stress was detected at the position of (a) as shown in FIG. 21 after the blade reached the crystal body. Thereafter, the stress increased substantially in proportion to the displacement amount of the blade. Phase transition did not occur in this process, but the daughter phase was generated in the mother phase due to phase transition at the position of (b). As the displacement amount of the blade increased, the phase boundaries grew in the direction of applying the shear force 4. This resulted in a structure, at the position (d), in which a mother phase, daughter phase and mother phase were arranged in this order in the direction perpendicular to the direction of applying the shear force 4. When the displacement amount of the blade was further increased from the position of (d), the daughter phase grew in that structure. That is, in this process, the transition from the mother phase to the daughter phase progressed in the direction perpendicular to the direction of applying the shear force 4.
(e) in FIG. 21 is the switching point for the unloading process (pulling back the blade), and the phase change of the daughter phase into the mother phase progressed in the direction perpendicular to the direction of applying the shear force 4 to the position of (f) after the unloading. Thereafter, from the position of (f) to the position of (g), the phase change from the daughter phase into the mother phase progressed in the direction of applying the shear force 4. The daughter phase then vanished at the position (g).

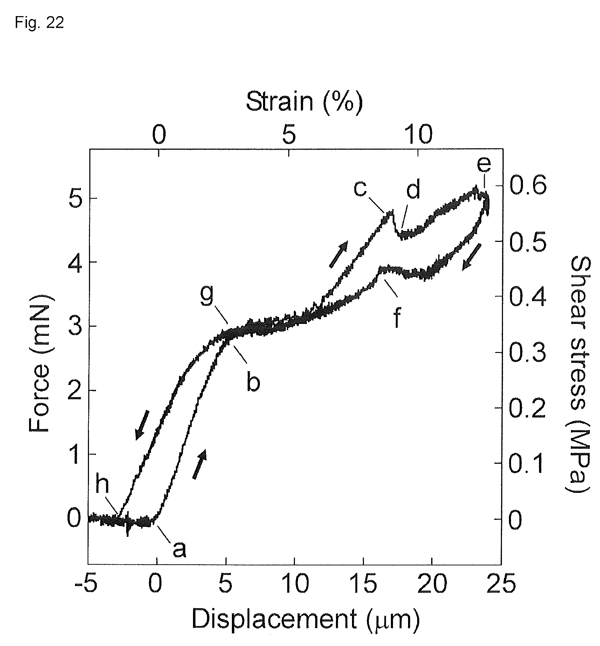

The shear stress curve thus obtained (FIG. 22) clearly demonstrated that the crystal body according to the present example exhibited superelasticity within a range from the position of (b) to the position of (g) via the position of (e).

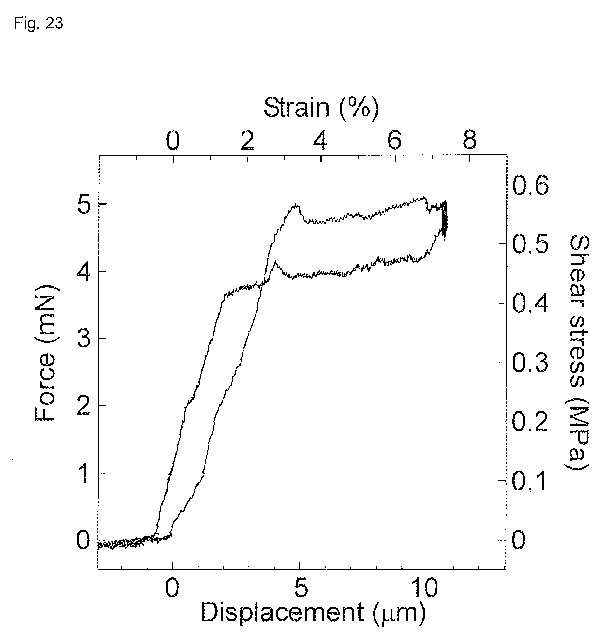

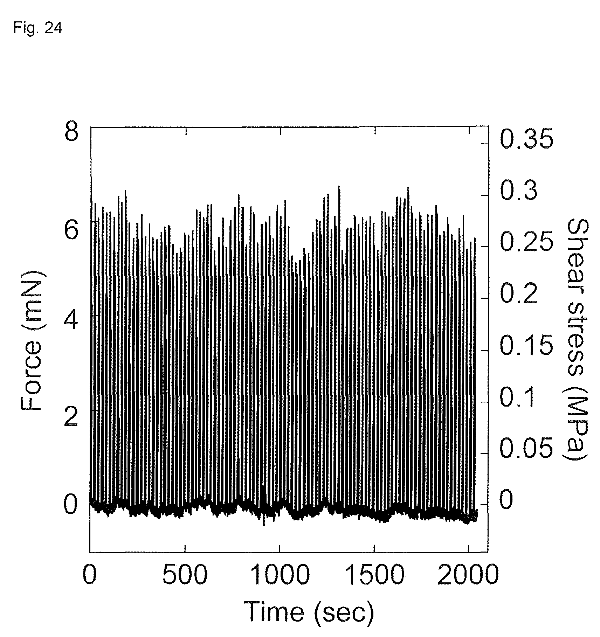

Here, the shear stress curve shown in FIG. 22 was dominantly affected by the effects, such as due to deformation of a holding member for fixing and supporting the crystal body, within a range from the position of (a) at which the blade came into contact with the crystal body to the position of (b) and within a range from the position of (g) to the position of (h) at which the blade was pulled away from the crystal body. Therefore, after obtaining a state of eliminating the effects, such as due to deformation of the holding member, the shear stress curve of the crystal body according to the present example was measured under an environment of 399.7K (FIG. 23). In addition, as shown in FIG. 24, the above superelastic transformation in the crystal body according to the present example was reproduced in 50 cycles without elastic reduction or material deterioration.

On the basis of the shear stress curve shown in FIG. 23, the energy storage density of the superelastic material formed of the crystal body according to the present example was estimated to be 26.5 kJm⁻³, and the energy storage efficiency (η) was estimated to be 0.766. Similarly on the basis of the shear stress curve shown in FIG. 23, the chemical stress was estimated to be 0.472 MPa, and the superelastic index of the superelastic material was estimated to be about 0.0561.

### (Example 5)

The relationship between the shear force and deformation of a crystal body formed of the single crystal of (PⁿBu₄) (BPh₄) was further investigated by applying a load to a part of the {0-10} crystal surface in the same manner as in Example 4 and varying the ambient temperature.

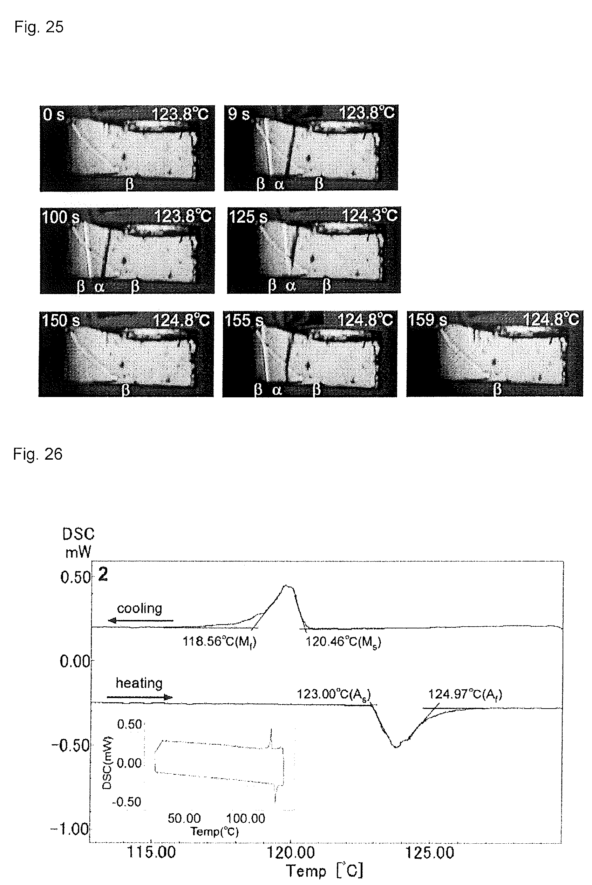

First, the ambient temperature was set to 397K (123.8°C) so that the crystal body would consist of the β-phase as a whole ("0 s" in FIG. 25). This state will be referred to as an "initial state." The shear force 4 was then applied to generate the α-phase to obtain a β/α two-phase coexisting state ("9 s" in FIG. 25). Subsequently, unloading was performed and it was confirmed that, even after 100 seconds passed from the initial state (i.e. even after 90 seconds or more passed from the unloading), the deformation strain due to applying the shear force 4 was maintained ("100 s" in FIG. 25).

During the period (50 seconds) from when 100 seconds passed to when 150 seconds passed after the initial state, the ambient temperature was raised from 397K (123.8°C) to 398K (124.8°C). As shown in "125 s" of FIG. 25, the crystal body when the ambient temperature was 397.5K (124.3°C) had a reduced volume of the α-phase compared with that of the crystal body when the ambient temperature was 397K (123.8°C). In the state after 150 seconds passed from the initial state, the α-phase completely vanished and only the β-phase existed ("150 s" in FIG. 25).

Thereafter, during the period to when 159 seconds passed from the initial state, the ambient temperature was maintained at 398K (124.8°C), and applying the shear force 4 and unloading were performed. As a result, it was confirmed that applying the shear force 4 allowed the α-phase to be formed in the crystal body of the β-phase ("155 s" in FIG. 25) and unloading the shear force 4 allowed the α-phase in the crystal body to vanish ("159 s" in FIG. 25).

For reference, a DSC (differential scanning calorimetry) chart of (PⁿBu₄) (BPh₄) is shown in FIG. 26, and the measurement results are listed in Table 4.

**[Table 4]**

| Crystal | Phase transition (α to β) | | | Melting (fusion of β crystal) | | | Index temperature | | |
|---|---|---|---|---|---|---|---|---|---|
| | T_{α-β}/°C | ΔH/kJmol⁻¹ | ΔS/ JK⁻¹mol⁻¹ | Tₘ/°C | ΔH/kJmol⁻¹ | ΔS/JK⁻¹mol⁻¹ | Aₛ(A_{f})/ °C | Mₛ(M_{f})/°C | Aₛ-Mₛ |
| (PBu₄)(BPh₄) | 122.9 | 3.01 | 7.6 | 230.1 | 41 | 81.4 | 123 (124.97) | 120.46 (118.55) | 2.54 |

### (Example 6)

The relationship between the shear force and deformation of a crystal body formed of the single crystal of (PⁿBu₄) (BPh₄) was further investigated by applying a load (shear force 4) to a part of the {-101} crystal surface of the α-phase, which is a crystal surface corresponding to the {0-10} crystal surface of the β-phase, in the same manner as in Example 4, and varying the ambient temperature in a different profile from that for Example 5.

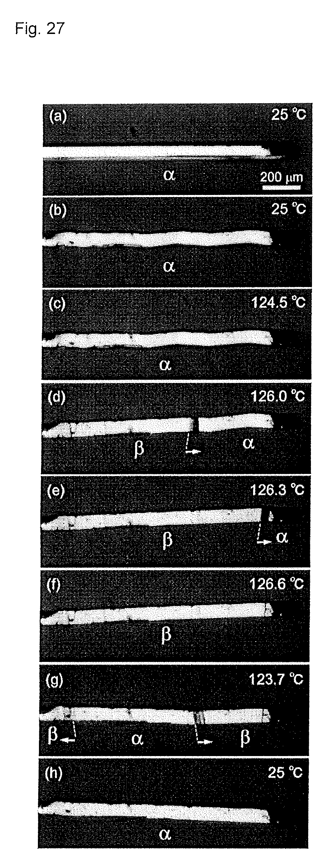

First, the ambient temperature was set to 298K (25°C) so that the crystal body would consist of the α-phase as a whole (FIG. 27(a)). When the shear force 4 was then applied, the deformation strain of the α-phase due to applying the shear force 4 was maintained (FIG. 27(b)).

Subsequently, the crystal body as a whole was heated by raising the ambient temperature. As a result, the α-phase was maintained with the deformation strain until the temperature reached 397.5K (124.5°C) (FIG. 27(c)) as in FIG. 27(b). When the ambient temperature exceeded 398K (125°C), the β-phase was generated in the crystal body as shown in FIG. 27(d), and the phage change of the α-phase into the β-phase rapidly progressed. As the phase change progressed, the deformation strain maintained in the crystal body decreased (FIG. 27(e)). When the ambient temperature was approximately 400K (126.6°C), the phase change of the α-phase as a low-temperature phase into the β-phase as a high-temperature phase was completed in the crystal body as shown in FIG. 27(f), and the deformation strain maintained in the crystal body completely vanished. From the above observation, the shape restoration (shape memory effect) due to temperature raise was confirmed.

The ambient temperature was then reduced to cool the crystal body of the β-phase. This resulted in the phase change of the β-phase into the α-phase when the ambient temperature was about 397K (124 °C), and this phase change rapidly progressed (FIG. 27 (g)). When the ambient temperature was further reduced, the crystal body was caused to consist only of the α-phase as a low-temperature phase without deformation strain, and when the ambient temperature was room temperature (25°C), the crystal body was restored to have the same phase state and shape as those in FIG. 27(a).

As described above, the crystal shape restoration cycle due to heat was confirmed from the present example.

### (Example 7)

Applying a shear force to the {0-10} crystal surface of the crystal body and unloading were performed in different temperature environments to obtain a shear stress curve at each temperature. From each shear stress curve, the energy storage density (or energy density), energy storage efficiency (η) (or energy efficiency) and superelastic index were obtained. Results are listed in Table 5.

**[Table 5]**

| Temperature (K) | Energy density (kJ m⁻³) | Energy efficiency | Superelastic index |
|---|---|---|---|
| 396.7 | 3.68 | 0.0556 | 0.0681 |
| 397.2 | 9.19 | 0.333 | 0.0808 |
| 397.7 | 14.2 | 0.5 | 0.0832 |
| 398.2 | 18.9 | 0.536 | 0.0773 |
| 398.7 | 21.6 | 0.629 | 0.0666 |
| 399.2 | 21.9 | 0.756 | 0.0562 |
| 399.7 | 26.5 | 0.766 | 0.0561 |

### (Example 8)

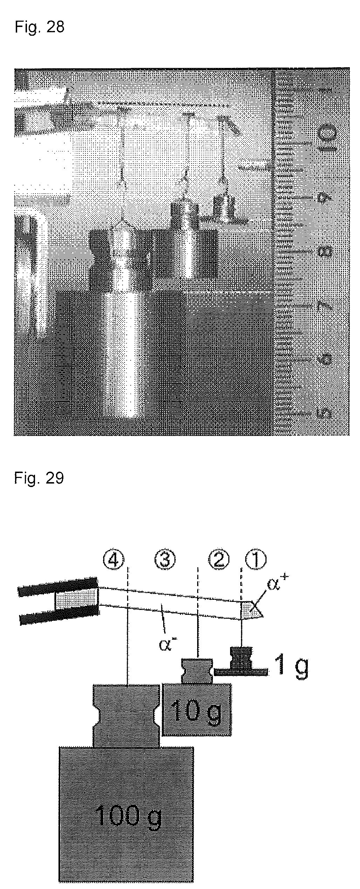

A single crystal of (PⁿBu₄) (BPh₄) was obtained in the same manner as in Example 4. The dimension was 3 mm×6 mm×34 mm, and the mass was 0. 61 g. In a room temperature environment (25°C), the single crystal of (PⁿBu₄) (BPh₄) was supported so that the center of the single crystal was projected with a distance of 30 mm. A weight of 100g was hung from a position of 5 mm as the distance from the supporting point using a string, a weight of 10g was hung from a position of 20 mm using a string, and a weight of 1g was hung from a position of 25mm using a string. In this state, the portion from the supporting point to the hanging position of the 1-g weight was an α₋-phase (daughter phase), and the other portion was an a₊-phase (mother phase) (FIGS. 29 and 29).

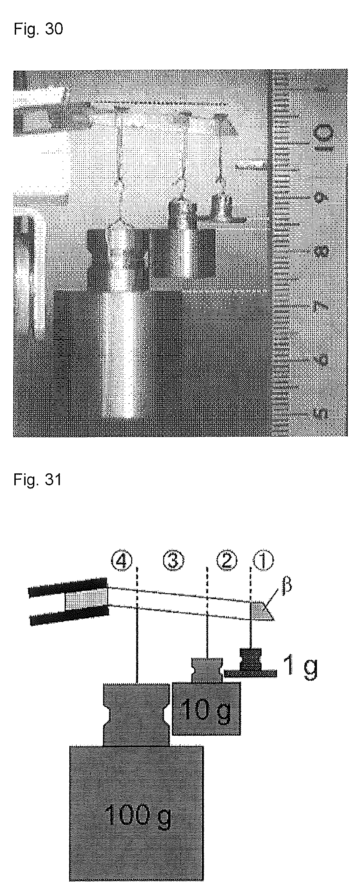

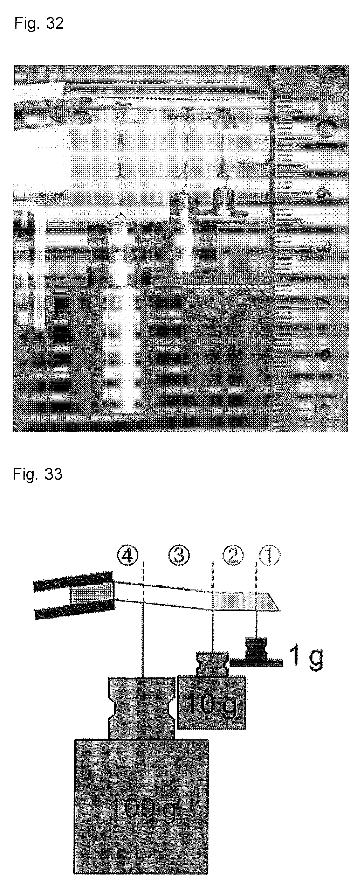

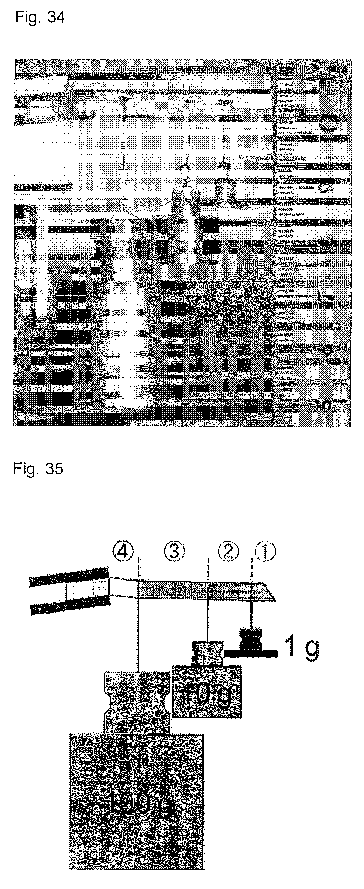

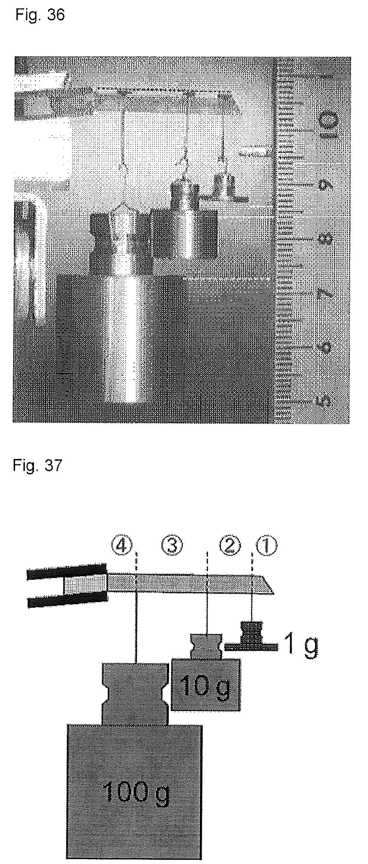

In this state, the ambient was maintained at 404.2K (131°C). As a result, the region of the single crystal from the end to the hanging position of the 1-g weight was transformed to the β-phase (FIGS. 30 and 31). Thereafter, when the ambient temperature was raised at a rate of 7 × 10⁻³Ks⁻¹, the β-phase of the single crystal grew toward the supporting point, and the 1-g weight continued to be raised. When the ambient was 405K (131.8°C), the β-phase further grew from the end to the hanging position of the 10-g weight in the single crystal, and the 10-g weight started to be raised (FIGS. 32 and 33). The temperature was further raised at a rate of 7 × 10⁻³Ks⁻¹ to grow the αβ-phase. When the ambient temperature was 406.6K (133.4°C), the 100-g weight started to be raised (FIGS. 34 and 35). At 409.2K (136°C), the single crystal became the β-phase up to the supporting point (FIGS. 36 and 37).

## Claims

1. A superelastic material having a molecular crystal.

2. The superelastic material as recited in claim 1, wherein the molecular crystal has an organic skeleton.

3. The superelastic material as recited in claim 1 or 2, wherein the superelastic material is formed of a molecular crystal body.

4. The superelastic material as recited in claim 1 or 2, wherein the superelastic material is formed of a mixture that includes a molecular crystal body.

5. The superelastic material as recited in claim 3 or 4, wherein the molecular crystal body is a single-crystal body.

6. The superelastic material as recited in claim 3 or 4, wherein the molecular crystal body is a polycrystal body.

7. The superelastic material as recited in any one of claims 4 to 6, wherein the mixture includes the molecular crystal body and a matrix material.

8. The superelastic material as recited in any one of claims 1 to 7, wherein the superelastic material has an energy storage density of 1 MJm⁻³ or less.

9. An energy storage material comprising the superelastic material as recited in any one of claims 1 to 8.

10. An energy absorption material comprising the superelastic material as recited in any one of claims 1 to 8.

11. An elastic material comprising the superelastic material as recited in any one of claims 1 to 8.

12. An actuator comprising the superelastic material as recited in any one of claims 1 to 8.

13. A shape memory material comprising the superelastic material as recited in any one of claims 1 to 8.
